# FASCICULE DE BREVET EUROPEEN

(11) **EP 3 522 772 B1**
(45) Date de publication et mention de la délivrance du brevet: **09.03.2022**
(21) Numéro de dépôt: 17780375.6
(22) Date de dépôt: 02.10.2017
(51) Int. Cl.: A61B 5/00, A45D 44/00

(54) **SYSTEME D'ANALYSE DES PROPRIETES PHYSICO-CHIMIQUES D'UNE SURFACE CUTANEE**
SYSTEM ZUR ANALYSE DER PHYSIKALISCH-CHEMISCHEN EIGENSCHAFTEN EINER HAUTOBERFLÄCHE
SYSTEM FOR ANALYSING THE PHYSICO-CHEMICAL PROPERTIES OF A SKIN SURFACE

(30) Priorité: 04.10.2016 FR 1659545
(43) Date de publication de la demande: 14.08.2019
(73) Titulaire: IEVA, 75002 Paris (FR)
(72) Inventeur: TREGUER, Yann, 29460 Logonna-Daoulas (FR)
(74) Mandataire: Cabinet Laurent & Charras
(86) Numéro de dépôt international: PCT/EP2017/075012
(87) Numéro de publication internationale: WO 2018/065380

(56) Documents cités:
- US-A1- 2004 199 058
- US-A1- 2009 143 653
- US-A1- 2015 230 863
- US-A1- 2016 058 364
- US-A1- 2016 235 374

## Description

### Domaine technique

L'invention se rattache au domaine de la beauté incluant la cosmétologie et de la dermatologie ainsi que le soin à la personne (également appelé « Personal Care » dans la littérature anglo-saxonne), le luxe et le style de vie (également appelé « life style» dans la littérature anglo-saxonne). Elle vise plus particulièrement un système permettant d'analyser différents paramètres physico-chimiques d'une surface cutanée, de manière à permettre de faire une recommandation ou diagnostiquer un éventuel traitement, et le cas échéant d'indiquer quels produits de traitement seraient les plus appropriés.

### Techniques antérieures

Comme on le sait, la peau est un organe majeur de l'organisme. Elle permet entre autre : le maintien de la température corporelle, l'isolement du milieu corporel intérieur, la limitation des pertes d'eau, la protection aux rayons ultraviolets, tout en assurant la synthèse de vitamine D. Elle joue donc un rôle de protection physique de l'organisme, et est le siège de nombreux échanges entre l'organisme et le milieu extérieur.

Cette exposition au milieu extérieur peut provoquer des dégradations de ses propriétés mécanique et chimique, ainsi que de son aspect visuel. De nombreux facteurs, et notamment les conditions atmosphériques, ou bien encore des phénomènes de pollution peuvent provoquer une modification de la structure de la peau et une dégradation de ces fonctions essentielles.

Actuellement les particules fines menacent la santé de millions d'humains dans le monde. Outre l'augmentation du nombre de cancers et de maladies pulmonaires et cardiovasculaires, ces particules ont aussi un fort impact sur le vieillissement de la peau notamment par la génération de stress oxydatif.

Les particules fines sont particulièrement dangereuses à cause de leur petite taille combinée à leur large surface par unité de masse, les rendant ainsi très réactive avec les structures biologiques. De plus, ces particules sont capables de transporter des molécules chimiques dans les mitochondries et ainsi de générer des dérivés réactifs de l'oxygène. C'est le cas notamment des hydrocarbures polycycliques aromatiques, qui se transforment en quinones, responsable de la production de réactif de l'oxygène. Les hydrocarbures polycycliques aromatiques sont aussi capables de déclencher la prolifération des mélanocytes et donc une coloration de la peau.

Une étude menée sur 400 femmes a montré l'impact de la pollution aux particules fines sur la peau. Cette étude a montré une corrélation entre l'exposition aux particules fines et les signes de vieillissement cutané. De plus, le pli nasogénien est plus prononcé chez les personnes soumises à ces particules.

Outre les particules fines, le milieu extérieur peut également être pollué par une sur concentration d'ozone, de dioxyde de soufre ou de dioxyde d'azote. Les conditions climatiques ont également un impact majeur sur la peau. A titre d'exemple, une trop forte exposition au rayonnement ultraviolet du soleil, ou à une atmosphère trop faiblement chargée en humidité peuvent provoquer une modification du métabolisme des cellules de la peau, et par exemple un vieillissement accéléré. D'autres facteurs peuvent également altérer la peau, tels que la lumière, le bruit ou les fumées de cigarette et de gaz d'échappement.

On observe alors des phénomènes de dessèchement de la peau, ou bien encore l'apparition de rides ou ridules. Jusqu'à présent, le diagnostic d'un traitement destiné à réduire l'importance de ces rides s'effectue par un examen visuel des zones cutanées à traiter. Aucune quantification précise de l'importance de ces rides ne peut être réalisée de façon rapide et généralisée.

On connaît l'existence de machines ou d'appareils sophistiqués incluant des dispositifs de prise de vue microscopiques permettant de visualiser la forme et les différentes dimensions d'un sillon de rides. De telles machines sont extrêmement complexes, et n'existent qu'en nombre très limité, ce qui ne permet pas leur emploi de façon suffisamment répandue. De plus, de tels appareils permettent uniquement de quantifier les symptômes et ne considèrent pas les causes de ces derniers. Le simple traitement des symptômes n'est pas toujours suffisant.

Par ailleurs, le traitement de la sécheresse cutanée ou d'une teneur trop forte en lipides est généralement diagnostiqué par une observation visuelle, voire un examen tactile de la peau. On perçoit les limites d'un tel examen qui, ne permet pas d'appréhender plusieurs symptômes simultanément. Or, il est reconnu que différents facteurs, tel qu'un faible taux d'humidité de la peau et un fort taux lipidique peuvent interférer et conduire à des erreurs de diagnostic. Or, un traitement qui n'est pas approprié peut avoir tendance à accentuer les défauts qu'il cherche à compenser.

Le but de la présente invention est donc de faciliter le diagnostic du traitement cutané par une analyse objective et rigoureuse des propriétés physico-chimiques de la peau.

Les documents FR 2 603 183 et JP 10 234676 décrivent des appareils de mesure des propriétés de la peau qui utilisent des capteurs spécifiques. De tels appareils comportent une ou plusieurs sondes différentes qui sont reliées une unité de traitement électronique. Les signaux générés par ces différentes sondes sont analysés par rapport à des seuils prédéterminés pour indiquer la position des valeurs mesurées par rapport aux seuils prédéterminées.

On conçoit que les analyses effectuées par ce type d'appareil ne sont pas réellement satisfaisantes, puisqu'elles ne prennent pas en compte les influences des différents paramètres de la peau les uns sur les autres, avec donc des risques d'erreurs de diagnostic.

En outre, si l'on veut effectuer la mesure de plusieurs paramètres relatifs à une zone localisée de la peau, l'emploi de plusieurs sondes distinctes nécessite de positionner successivement les différentes sondes au même endroit de la peau, avec des risques d'erreurs, et une durée importante de l'opération.

Le brevet européen EP 1 439 782 propose de remédier à ce problème par l'utilisation d'un dispositif comportant un ensemble de sondes. Bien que ce dispositif permette une analyse précise et homogène de plusieurs paramètres de la peau, sur une même zone localisée de la peau, il ne permet d'effectuer qu'une mesure ponctuelle, c'est-à-dire à un instant précis, des caractéristiques physico-chimiques de la peau.

Le document US 2009/143653 divulgue un dispositif d'analyse des propriétés physico-chimiques d'une surface cutanée se présentant sous la forme d'une carte à puce et comportant: au moins un capteur apte à délivrer un signal représentatif d'une mesure de la propriété physico-chimique; une unité de traitement apte à conditionner le signal délivré par le capteur et à le mémoriser dans une unité de stockage; une source d'énergie électrique pour alimenter ledit capteur et l'unité de traitement.

L'invention vise à améliorer la qualité de l'estimation des caractéristiques physico-chimiques de la peau.

### Exposé de l'invention

L'invention propose de résoudre ce problème technique en utilisant au moins un capteur d'environnement configuré pour accompagner l'utilisateur au cours de la journée afin de mesurer les conditions dans lesquelles évolue la peau de l'utilisateur. L'estimation des caractéristiques physico-chimiques de la peau est ainsi effectuée à partir des mesures ponctuelles de la peau mais également à partir des mesures au cours du temps de l'au moins un capteur d'environnement.

Au sens de l'invention, une mesure ponctuelle correspond à une mesure effectuée manuellement par l'utilisateur avec des intervalles de temps non prédictifs et variables. Par exemple, une mesure ponctuelle est effectuée une à deux fois par jour, le matin et/ou le soir, à des heures variables.

Au sens de l'invention, une mesure au cours du temps correspond à une mesure effectuée automatiquement avec des intervalles de temps sensiblement réguliers.

Par exemple, une mesure au cours du temps peut être effectuée entre 9h et 18h toutes les 10 min. Selon un autre exemple, l'intervalle de temps peut varier automatiquement en fonction de la différence entre une mesure courante et une mesure précédente. Lorsque la différence entre deux mesures est faible, le temps entre deux mesures peut être augmenté alors que, lorsque la différence entre deux mesures est importante, le temps entre deux mesures peut être diminué.

Par surface cutanée, on sous-entend bien entendu l'ensemble de l'enveloppe cutanée, y compris les zones pileuses, et notamment le cuir chevelu.

L'invention concerne donc un système d'analyse des propriétés physico-chimiques d'une surface cutanée, défini par la revendication indépendante 1.

L'invention permet ainsi d'estimer les paramètres physiologiques de la peau en croisant une mesure ponctuelle avec une mesure des sollicitations externes subies par la peau au cours de la journée. Autrement dit, le système conforme à l'invention permet à l'utilisateur de déterminer simultanément plusieurs informations de natures différentes, relatives à une zone particulière de sa surface cutanée et aux sollicitations subies par cette surface au cours de la journée.

L'ensemble de ces informations peut ensuite être décodé de manière à déterminer chacune des propriétés physico-chimiques de la surface cutanée qui présente un intérêt en vue du traitement à venir.

De préférence, l'au moins un capteur de contact est configuré pour mesurer un taux d'hydratation et/ou une quantité de sébum et/ou un taux de desquamation.

De préférence, le système intègre une pluralité de capteurs de contact rassemblés dans une zone restreinte, ce qui permet d'obtenir des résultats représentatifs de la même zone pour tous les paramètres analysés.

Par exemple, lorsque le capteur de contact est configuré pour mesurer uniquement un taux d'hydratation, une solution classique consister à conseiller une crème hydratante lorsque le taux d'hydratation mesuré est inférieur à une valeur seuil. L'invention permet de nuancer cette mesure en interprétant les conditions subies par la peau au cours de la journée. Par exemple si une température extérieure mesurée par le capteur d'environnement était inférieure à -10° durant plusieurs heures de la journée, il est possible que la peau soit déshydratée uniquement par le contact avec le froid. L'invention permet ainsi de conseiller une crème de régénération de la peau au lieu d'une crème hydratante.

De façon préférée, les différents capteurs de contact et d'environnement sont réalisés par des technologies de type MEMS, signifiant "Système Microélectromécanique". Ces capteurs de contact et d'environnement sont donc réalisés selon des technologies utilisant des matériaux semi-conducteurs, isolants ou conducteurs, et des méthodes d'usinage chimiques employés dans le domaine de la microélectronique. L'emploi de capteurs de contact de type MEMS permet de concentrer l'ensemble des capteurs de contact sur une zone particulièrement restreinte, implantée sur une sonde unique.

De préférence, l'au moins un capteur d'environnement est configuré pour mesurer une température et/ou une information climatique et/ou une information relative à une pollution environnementale.

Au sens de l'invention, une mesure d'une information climatique correspond à une information sur le taux d'humidité dans l'air, sur la pression atmosphérique, sur la vitesse du vent, sur la quantité de rayons ultraviolets reçue par la peau, sur la température, sur la température ressentie (combinaison de la température, du taux d'humidité et des mouvements d'air), ou un ensemble de ces informations.

Au sens de l'invention, une mesure d'une information relative à une pollution environnementale correspond à une mesure des particules fines en suspension, du dioxyde de soufre, du dioxyde d'azote, de l'ozone, du benzène, des métaux lourds comme le plomb ou le mercure, d'hydrocarbures, du monoxyde de carbone ou d'autres composés volatils organiques, ou un ensemble de ces informations.

En outre, une information relative à une pollution environnementale peut également concerner une pollution sonore pouvant appartenir aux infra-sons, à la bande spectrale audible et aux ultra-sons, car certaines fréquences peuvent être génératrices de stress pour la peau. Par exemple, pour la bande spectrale audible, un microphone tel que ceux intégrés dans un téléphone portable peut être utilisé pour réaliser la fonction d'un capteur d'environnement.

En outre, une information relative à une pollution environnementale peut également concerner une luminosité. En effet, la luminosité joue un rôle de régulateur de l'horloge biologique du corps par le biais de l'hypothalamus. Celuici contrôle le système nerveux et le système endocrinien qui, ensemble, régulent toutes les fonctions biologiques du corps humain. De plus, l'hypothalamus supervise les informations liées à la lumière et les envoie au corps pinéal, lequel les utilise pour informer les autres organes, comme la peau, des conditions lumineuses de l'environnement.

Tous les êtres vivants sont soumis à des rythmes biologiques, c'est-à-dire à des phénomènes biologiques qui se répètent à intervalles réguliers dans le temps. La préservation de ces rythmes est essentielle car elle constitue une des composantes essentielles de notre bonne santé. Le rythme circadien, d'une durée de 24 heures, permet aux organismes de s'adapter à la périodicité de l'alternance jour/nuit. Il concerne non seulement l'alternance veille/sommeil, mais aussi d'autres paramètres physiologiques, comme la température corporelle, la circulation sanguine, la production d'urine, etc. Lorsque l'horloge biologique n'est pas en phase avec les signaux extérieurs majeurs, il en résulte un dysfonctionnement qui conduit à la désynchronisation du système circadien de l'organisme. Cette désynchronisation s'accompagne souvent de signes inhabituels comme une fatigue persistante, des troubles du sommeil, une diminution de l'attention, voire même dans les cas extrêmes une dépression ou des insomnies.

Selon un mode de réalisation, la mesure au cours du temps d'au moins un paramètre extérieur agissant sur ladite surface cutanée est effectuée par une mesure de la position du capteur d'environnement couplée à une information relative à une information climatique déterminée en fonction de la position dudit capteur d'environnement. Ce mode de réalisation permet d'interroger un serveur contenant une pluralité de données environnementales en envoyant uniquement la position de l'utilisateur.

Selon un mode de réalisation, l'au moins un capteur de contact intègre des moyens de communication sans fil avec ladite unité de traitement. Autrement dit, les signaux générés par le capteur de contact sont transmis à l'unité de traitement, avec éventuellement un premier traitement de mise en forme, par une liaison de type hertzien. Cette disposition confère plus de souplesse de manipulation, puisqu'il est ainsi possible de déplacer le capteur de contact dans l'espace, sur différentes zones cutanée d'un même patient, ou au sein du local dans lequel est disposé l'appareil, en étant uniquement limité par des considérations de portée de la liaison sans fil. En pratique, la liaison peut fonctionner par exemple selon la technologie connue sous l'appellation "Bluetooth".

On pourra notamment utiliser la bande de fréquence dédiée aux applications industrielles, également connue sous l'abréviation ISM Band pour "Industrial Scientific Medical Band" ou encore les réseaux mobiles définis par la norme LTE regroupant les normes GSM a UMTS (ou plus communément appelée « 3G » et « 4G ») ou d'autre réseaux de télécommunications dédiés aux systèmes connectés.

Selon un mode de réalisation, l'au moins un capteur d'environnement intègre des moyens de communication sans fil avec ladite unité de traitement. Ce mode de réalisation permet de transporter facilement le capteur d'environnement, par exemple sous la forme d'une broche ou d'une montre connectée.

Selon un mode de réalisation, les capteurs de contact et d'environnement sont incorporés dans un même boîtier. Ce mode de réalisation permet à l'utilisateur de transporter le capteur de contact afin de réaliser un test de la surface cutanée à tout moment de la journée.

Selon un mode de réalisation, l'unité de traitement est intégrée dans un téléphone intelligent. Ce mode de réalisation permet d'analyser les informations reçues par les capteurs de contact et d'environnement et d'afficher les conseils de traitement de la surface cutanée en temps réel ainsi qu'en utilisation nomade.

En variante, l'unité de traitement peut être interfacée avec un serveur distant qui effectue l'analyse. Pour ce faire, le téléphone intelligent communique les informations issues des capteurs de contact et d'environnement de manière sécurisé avec un serveur stockant les précédentes informations de l'utilisateur. Le serveur peut ensuite transmettre au téléphone intelligent les recommandations en fonction des informations reçues mais également des informations précédentes.

Selon un mode de réalisation, l'unité de traitement effectue un classement de la surface cutanée à analyser dans une catégorie prédéterminée en fonction des propriétés physico-chimiques déterminées.

Ce classement de la peau permet d'associer un traitement préventif ou correctif en fonction de la catégorie de la peau.

Selon un mode de réalisation, l'unité de traitement effectue une détection d'un produit à conseiller en fonction de la catégorie prédéterminée et d'une base de données de produits traitants. Cette détection permet de proposer un produit particulièrement efficace et répondant aux besoins de la peau.

### Description sommaire des figures

La manière de réaliser l'invention ainsi que les avantages qui en découlent ressortiront bien de la description du mode de réalisation qui suit, à l'appui des figures annexées dans lesquelles :
la figure 1 est une représentation schématique d'un système d'analyse des propriétés physico-chimiques d'une surface cutanée selon un mode de réalisation de l'invention ; et
la figure 2 est une représentation schématique du processus de conseil d'un produit à appliquer sur la peau en fonction des informations issues des capteurs de contact et d'environnement du mode de réalisation de la figure 1.

### Manière de réaliser l'invention

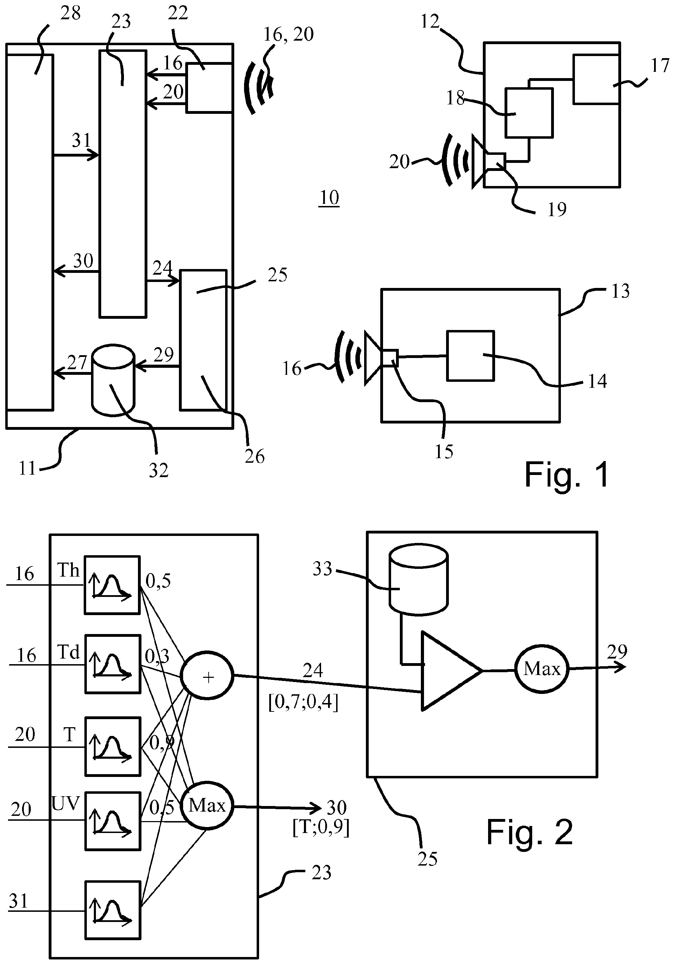

L'invention illustrée sur la figure 1 concerne un système **10** d'analyse des propriétés physico-chimiques d'une surface cutanée. Le système **10** comporte une unité de traitement **11** associée à un capteur de contact **12** et à un capteur d'environnement **13.**

Plus précisément, le capteur de contact **12** présente un ensemble de sondes **17** nécessaires à l'analyse des caractéristiques physico-chimiques de la surface cutanée sur laquelle est posé le capteur de contact **12.** De préférence, les sondes **17** mises en œuvre permettent de mesurer un taux d'hydratation et/ou une quantité de sébum et/ou un taux de desquamation.

Par exemple, un capteur d'hydratation, basé sur la technologie MEMS, mesure une variation de capacitance entre l'air et la surface cutanée en contact avec ce dernier. En effet, la constante diélectrique de la peau est proportionnelle à la quantité d'eau qu'elle contient. Cette méthode de mesure permet de contrôler la pénétration des lignes de champs électrostatiques par la géométrie du capteur (peignes inter-digités) et par la fréquence d'excitations. De plus, ce procédé est non invasif et la rapidité d'acquisition, inférieur à 5 secondes, permet de s'abroger des réactions de la peau au contact du capteur.

Pour mesurer la densité de sébum, deux méthodes peuvent être utilisées. Une première méthode consiste dans l'utilisation des propriétés de fluorescence du sébum. Sous un rayonnement bleu profond / proche UV-A, le sébum réagit en émettant un rayonnement autour des 560nm (Orange-rouge). Pour ce faire une source lumineuse monochromatique (ou à bande spectrale étroite) est utilisée centrée autour des 395nm. L'acquisition de la fluorescence du sébum est assurée par l'utilisation d'un capteur CMOS interfacé avec un filtre passe-haut avec une fréquence de coupure autour des 510nm. L'image obtenue est segmentée par des algorithmes de traitements d'image pour quantifier la surface couverte par le sébum. Une seconde méthode consiste dans l'utilisation d'un patch régissant au sébum (en devenant translucide au contact de lipide). Ce patch est appliqué sur la peau et capturé par un capteur CMOS sous un éclairage blanc avec une polarisation croisée (entre l'émission et le capteur CMOS) obtenue en utilisant des films polarisant linéaires. L'image obtenue est segmentée par des algorithmes de traitements d'image pour quantifier la surface couverte par le sébum.

Pour mesurer la desquamation, il est possible d'utiliser un patch adhésif permettant de recueillir les cellules mortes en l'appliquant sur la peau. Ce patch est ensuite capturé par un capteur CMOS sous un éclairage blanc avec une polarisation croisée puis avec une polarisation parallèle. Les deux images ainsi obtenues permettent de segmenter les cellules mortes suivant leur épaisseur et de déterminer un indice de desquamation.

Dans l'exemple de la figure 1, les informations **20** issues de ces sondes sont mises en forme par un composant **18,** par exemple un microcontrôleur, avant d'être transmises à l'unité de traitement **11** par un module de communication sans fil **19.**

Le capteur de contact **12** peut prendre une pluralité de formes sans changer l'invention. Par exemple, le capteur de contact **12** peut prendre la forme d'une poire avec une première partie destinée à saisir le capteur de contact **12** et une seconde partie destinée à mettre les sondes **17** au contact de la peau de l'utilisateur. Un bouton d'activation permet de déclencher une mesure **17** des paramètres de la peau de l'utilisation. En variante, le capteur de contact **12** peut être connecté à l'unité de traitement **11** par une liaison filaire sans changer l'invention.

Le capteur d'environnement **13** constitue également un objet nomade connecté à l'unité de traitement **11** par un module de communication sans fil **15.** Plus précisément, le capteur d'environnement **13** présente un ensemble de sondes **14** nécessaires à l'analyse de l'environnement dans lequel évolue l'utilisateur au cours de la journée. Par exemple, une sonde **14** peut être un simple capteur de température. D'autres sondes **14** plus complexes peuvent également être utilisées, telles que les sondes aptes à détecter la qualité de l'air.

Par exemple, la société AIRPARIF^{®} propose des sondes **14** permettant de détecter les particules fines. Les Matières particulaires, également connues comme "Particulate matter" ou PM, sont un mélange complexe de particules extrêmement petites et de gouttelettes liquides.

La pollution particulaire est constituée d'un certain nombre de composants, y compris des acides (par exemple les nitrates et les sulfates), des produits chimiques organiques, des métaux, et des particules de sol ou de la poussière. La pollution aux particules serait à l'origine de 42 000 morts prématurées par an en France et de nombreuses maladies (asthme, allergies, maladies respiratoires et cardiovasculaires, cancer du poumon).

Les plus grossières (supérieures à 2.5 micromètres) retombent assez vite, leur durée de séjour dans l'air est de l'ordre de 1 jour, tandis que les plus fines peuvent rester jusqu'à 1 semaine en suspension et parcourir des milliers de kilomètres. Une fois déposées, les particules peuvent ensuite être remises en suspension sous l'action du vent ou en zone urbaine, sous l'action du trafic routier.

La taille des particules est directement liée à leur dangerosité potentielle vis-à-vis de la santé. Les organismes environnementaux sont préoccupés par les particules qui ont un diamètre inférieur ou égale 10 micromètres parce que ce sont les particules qui passent généralement par le biais de la gorge et du nez et pénètrent dans les poumons. Une fois inhalées, ces particules peuvent affecter le cœur et les poumons et causer des effets graves sur la santé.

Les particules sont classées en quatre catégories:
- **PM 10,** particules grossières inhalables telles que celles trouvées près des routes et des industries poussiéreuses, elles sont inférieures à 10 micromètres de diamètre et incluent les particules fines, très fines et ultrafines.
- **PM 2.5,** particules fines telles que celles contenues dans la fumée et la brume, sont inférieures ou égales à 2,5 micromètres de diamètre. Ces particules peuvent être émises directement à partir de sources telles que les incendies de forêt, ou elles peuvent se former lorsque des gaz, émis par des centrales thermiques, des industries et des automobiles réagissent dans l'air. Les moteurs diésel en sont la source principale. Les particules fines incluent également les particules très fines et ultrafines.
- **PM 1,** particules très fines (les plus dangereuses pour la santé) sont inférieure ou égales à 1 micromètre de diamètre. Elles ne sont pratiquement éliminées que par les précipitations et ont le temps de s'accumuler dans l'air. Elles incluent ainsi les particules ultrafines.
- **PM 0.1,** particules ultrafines dont le diamètre est inférieur à 0,1 micromètre, appelées également « nanoparticules » Leur durée de séjour est très courte, de l'ordre de quelques minutes à quelques heures.

Les PM2.5 et les PM1 peuvent descendre dans la partie la plus profonde (alvéolaire) des poumons lorsque les échanges gazeux se produisent entre l'air et le sang. Ce sont les particules les plus dangereuses parce que la partie alvéolaire des poumons n'a pas de moyens efficaces de les éliminer et si les particules sont solubles dans l'eau, elles peuvent passer dans le flux sanguin en quelques minutes. Si elles ne sont pas solubles dans l'eau, elles restent dans la partie alvéolaire des poumons pendant une longue période. Les éléments solubles peuvent être des HAP (Hydrocarbure Aromatique Polycyclique) ou des résidus de benzène classés comme cancérogène.

Les sondes **14** peuvent consister en un détecteur optique de particules. Le principe de fonctionnement de ces sondes **14** est le suivant : quand un faisceau laser traverse de l'air pur, le faisceau est invisible. Lorsque le faisceau est visible, c'est parce que le faisceau se diffracte sur des particules tout au long de son chemin. Une telle sonde de particules utilise une source proche de l'infrarouge, telle qu'une diode laser à avalanche ou une diode électroluminescente avec un angle (ou faisceau) d'émission étroit associée à un amplificateur afin de détecter la visibilité du faisceau.

Chaque particule qui passe devant le faisceau laser diffracte une partie de ce faisceau vers la sonde et, le flux d'air étant constant, la largeur de l'impulsion mesurée permet de classer les particules par taille. Une moyenne glissante des quantités de particules par catégorie est réalisée sur une période de 30 secondes.

D'autres types de sondes peuvent également être utilisées telles que les compteurs à noyaux de condensation, les sondes de tailles de particules (ou APS pour « Aerodynamic Particle Sizer » dans la littérature anglo-saxonne), les analyseurs différentiels de mobilité électrique, les granulomètres DMPS, les échantillonneurs ELPI, ainsi que bien d'autres sondes basées sur les principes de détection par mesure de masse.

Le principe des Compteurs à Noyaux de Condensation (CNC) est de faire grossir artificiellement les particules par condensation d'eau ou de butanol de façon à pouvoir les détecter avec un système optique classique. Les CNC permettent de détecter les particules comprises entre 3 nm et 1,1 µm de diamètre.

L'APS (Aerodynamic Particle Sizer) permet de fournir une concentration en nombre de particules dans une tranche granulométrique de 0,5 µm à 20 µm. Le principe est celui de la spectrométrie de temps de vol. L'échantillon d'aérosols est accéléré dans un orifice. Le taux d'accélération des particules est déterminé par leur diamètre aérodynamique, les plus grosses ayant l'accélération la plus faible en raison d'une inertie plus forte. Après accélération, les particules traversent un système composé de deux faisceaux lasers, d'un miroir et d'un photodétecteur permettant de les dénombrer et de mesurer leur vitesse, et donc leur diamètre aérodynamique.

L'Analyseur Différentiel de Mobilité Electrique (ADME) ou « Differential Mobility Analyser » (DMA) dans la littérature anglo-saxonne, charge électriquement les particules, et les fait ensuite passer dans un champ électrostatique, l'ensemble permettant de faire sortir les particules à des moments différents en fonction de leur taille, la mobilité électrique étant inversement reliée à la dimension des particules. Le dénombrement des particules est ensuite effectué à l'aide d'un Compteur à Noyau de Condensation.

Le granulomètre DMPS (Differential Mobility Particles Sizer) ou SMPS (Scanning Mobility Particles Sizer) dans la littérature anglo-saxonne, associe ainsi un ADME et un CNC. Ce type d'appareil permet de connaître le nombre de particules entre 10 nm et 1 µm.

L'échantillonneur ELPI (Electrical Low Pressure Impactor) fonctionne sur le même principe que les impacteurs en cascade, mais les particules sont chargées en entrée de l'impacteur et un électromètre enregistre les charges induites sur chacun des étages lors de l'impaction des particules.

L'analyse du signal permet de caractériser la granulométrie, dans une gamme de 0,07 à 10 µm. Un programme d'acquisition permet de visualiser les distributions en nombre, en volume et en masse des particules.

Ainsi, le nombre de sondes **14** pouvant être utilisées pour mettre en œuvre l'invention est particulièrement important et permet d'obtenir des informations **16** très diverses sur les sollicitations subies par la peau au cours de la journée. La sonde **14** peut également réaliser une mesure de position. Cette mesure de position est alors transmise à l'unité de traitement **11** connectée à un serveur distant permettant d'associer, à la position de l'utilisateur, une information météorologique ou, plus généralement, une information relative à l'environnement.

Les mesures **16** des sondes **14** sont préférentiellement effectuées régulièrement au cours de la journée alors que le capteur d'environnement **13** est porté par l'utilisateur. Par exemple, le capteur d'environnement **13** peut comporter une horloge interne qui effectue des mesures **16** toutes les 10 min.

Le capteur d'environnement **13** peut prendre une pluralité de formes sans changer l'invention. De préférence, le capteur d'environnement **13** est un objet petit, c'est-à-dire dont les dimensions externes sont contenues dans une cube de 5cm de côté. Par exemple, le capteur d'environnement **13** peut être intégré dans un bracelet, un porte-clefs, un bijou de sac à main, une amulette ou une broche.

En outre, les capteurs **12-13** de contact et d'environnement peut être intégrés dans un seul et même boitier afin de limiter le nombre d'objets du système **10.**

Les mesures **16, 20** issues des capteurs **12-13** de contact et d'environnement sont ainsi transmises à l'unité de traitement **11** qui intègre plusieurs organes. Premièrement, ces mesures **16, 20** sont reçues par un récepteur sans fil **22** qui transmet ces mesures à des moyens d'analyse **23** permettant la détermination de certaines propriétés physico-chimiques **24** de la peau.

Un exemple de réalisation de ces moyens d'analyse **23** est illustré sur la figure 2. Deux mesures **16** issues du capteur de contact **12** et deux mesures **20** issues du capteur d'environnement **13** sont analysées par les moyens d'analyse **23.** La différence de chaque mesure **16, 20** est analysée avec une fonction gaussienne centrée sur la valeur moyenne attendue pour chaque mesure **16, 20.** La distance de la mesure **16, 20** avec la fonction gaussienne étant normalisée entre 0 et 1. Les distances sont ensuite corrélées pour obtenir un vecteur contenant les propriétés physico-chimiques **24** de la peau de l'utilisateur. En variante, les fonctions gaussiennes peuvent être remplacées par des tables de correspondance associant les propriétés de la peau aux paramètres environnementaux (en valeur absolue et en variation).

Par exemple, les deux mesures **20** issues du capteur de contact **12** peuvent représenter le taux d'hydratation **Th** et le taux de desquamation **Td** de la peau de l'utilisateur alors que les deux mesures **16** issues du capteur d'environnement peuvent représenter la température **T** et les rayonnements ultraviolets **UV** subies par la peau au cours de la journée. La distance entre le taux d'hydratation **Th** mesuré et le taux normal est de 0.5 à la sortie de la première fonction gaussienne et la distance entre le taux de desquamation **Td** de la peau et le taux normal est de 0.3 à la sortie de la deuxième fonction gaussienne.

La distance entre la température **T** mesurée au cours du temps et la résistance en température sur l'hydratation de la peau est de 0.9 en sortie de la troisième gaussienne et la distance entre les rayonnements ultraviolets **UV** subis par la peau et la résistance aux rayonnements sur le taux de desquamation est de 0.5 en sortie de la quatrième gaussienne. Il s'ensuit que le taux d'hydratation **Th** de la peau en sortie des moyens d'analyse **23** sera estimé à 0.7 comme étant la moyenne entre la distance entre le taux d'hydratation **Th** mesuré et le taux normal, 0.5, et la distance entre la température **T** mesurée au cours du temps et la résistance en température sur l'hydratation de la peau, 0.9.

De la même manière, le taux de desquamation **Td** de la peau en sortie des moyens d'analyse **23** sera estimé à 0.4. Le vecteur contenant les propriétés physico-chimiques **24** de la peau de l'utilisateur en sortie des moyens d'analyse **23** comprendra les valeurs [0.7 ; 0.4] selon cet exemple.

En outre, le maximum des distances des fonctions gaussiennes permet de détecter une propriété physico-chimique **30** de la peau ayant subie les plus grandes sollicitations. Dans l'exemple précédent, le maximum des distances est atteint pour la température **T** subie par la peau qui présente une distance de 0.9. Ainsi, le plus important facteur de stress de la peau sera estimé comme étant la température de l'environnement.

Le vecteur contenant les propriétés physico-chimiques **24** de la peau de l'utilisateur est ensuite transmis à un organe de classement **25** de la peau de l'utilisateur en comparant des vecteurs de références, stockés dans une base de données **33,** avec celui contenant les propriétés physico-chimiques **24** de la peau de l'utilisateur. Le maximum de corrélation entre les vecteurs de références et celui contenant les propriétés physico-chimiques **24** de la peau de l'utilisateur permet d'associer la peau de l'utilisateur à une catégorie **29.**

Un traitement **27** associé à cette catégorie **29** de peau est également stocké dans une base de données **32** de l'unité de traitement **11.** L'unité de traitement **11** comporte une interface utilisateur **28** affichant la propriété physico-chimique **30** de la peau ayant subie les plus grandes sollicitations ainsi que le traitement **27** proposé en fonction de la catégorie **29** de peau détectée.

Par exemple, si le taux d'hydratation a fortement diminué par rapport à une mesure précédente, par exemple deux jours avant, l'unité de traitement **11** va considérer précisément l'évolution des **16** issues du capteur d'environnement. Si la température **T** a fortement diminué par rapport aux deux jours précédents, il est possible de conclure que l'utilisateur a changé d'environnement, par exemple suite à un séjour au ski.

Ainsi, la chute de l'hydratation est normale et n'est pas liée à un problème physiologique. L'unité de traitement **11** proposera donc des produits à effet immédiat et non pas des produits traitants « sur le long terme ».

Selon un autre exemple d'utilisation des conditions environnementales sur les paramètres physico-chimiques de la peau, des variations importantes peuvent être constatées sur le taux d'hydratation en fonction de la température extérieure et du taux d'humidité relative. Si la mesure du taux d'hydratation est faite de façon ponctuelle sans prendre en compte les conditions environnementales, la valeur obtenue peut apparaître anormale alors qu'elle n'est en réalité que le reflet de l'environnement.

Un environnement tempéré, c'est-à-dire avec une température proche de 25°C et un taux d'humidité compris entre 60 et 65%, permet à la peau d'avoir un fonctionnement physiologique optimal. La mesure des paramètres physico-chimiques de la peau dans ces conditions sont alors représentatives de son état de santé. Lorsque le taux d'humidité relative est inférieur à 30%, le taux d'hydratation diminue car la perte insensible en eau augmente pour compenser la sécheresse de l'air. Ce phénomène est amplifié par les températures extrêmes, c'est-à-dire les températures inférieures à 5°C et supérieures à 29°C.

En ayant connaissance de ces conditions environnementales, le taux d'hydratation mesuré peut être interprété directement pour proposer un traitement temporaire adapté ; mais ce taux d'hydratation peut également être corrigé par la température ambiante et le taux d'humidité relative en utilisant une régression multilinéaire. Le taux d'hydratation corrigé est alors comparable à celui mesuré dans un environnement tempéré et un traitement à long terme peut être proposé. La détermination des paramètres de la régression multilinéaire est effectuée de manière empirique par des mesures sur un panel de personnes similaire.

En outre, l'interface utilisateur **28** peut permettre à l'utilisateur de rentrer certaines informations **31,** telles que le type de crèmes appliquées sur la peau de l'utilisateur au cours de la journée ou des contre-indications médicales à certains produits. Ce type d'information peut avoir un impact sur le classement de la peau de l'utilisateur et peut être utilisé par les moyens d'analyse **23.**

De préférence, l'unité de traitement **11** sera embarquée sur un téléphone intelligent afin d'utiliser le processeur et la mémoire du téléphone intelligent pour réaliser les traitements des mesures **16, 20.** En outre, les traitements peuvent être partiellement ou totalement déportés sur un serveur distant connecté au téléphone intelligent par une connexion sans fil de données.

Pour connaître les produits **27** à utiliser, l'utilisateur lance dans un premier temps l'application du téléphone intelligent et pourra retrouver les informations sur les mesures **16, 20** des journées précédentes. L'application peut également émettre des informations sur les facteurs de stress attendus pour la journée, par exemple les prévisions météorologiques.

Pour effectuer une mesure ponctuelle, l'utilisateur sera invité à utiliser le capteur de contact **12** et à le poser sur la surface à analyser, par exemple les pommettes du visage. Le début de mesure et la fin de mesure sont signalés par le téléphone intelligent par une vibration si le mode silence est actif ou par un son.

L'application pourra d'ores et déjà proposer des produits **27** en fonction de cette unique mesure. Si l'utilisateur envisage de sortir, l'utilisateur sera invité à emporter le capteur d'environnent **13** avec lui.

A l'issue de la journée, l'utilisateur pourra effectuer une nouvelle mesure ponctuelle au moyen du capteur de contact **12** et les résultats sont alors corrélés avec les mesures **16** captées au cours de la journée.

L'application peut contenir d'autres informations liées aux mesures **16, 20** effectuées permettant de guider l'utilisateur dans son style de vie afin d'améliorer la santé de sa peau. Par exemple, si la peau de l'utilisateur n'est pas en bonne santé et que les prévisions météorologiques annoncent des températures trop froides pour la peau, l'utilisateur pourra être conseillé de limiter son exposition durant quelques jours, le temps nécessaire pour reconstruire l'épiderme.

En outre, l'application comporte avantageusement une phase de configuration préalable à l'utilisation de l'application. Cette phase de configuration permet d'acquérir des informations de l'utilisateur afin de configurer l'application selon les besoins et les préférences de l'utilisateur. Par exemple, l'utilisateur est invité à répondre à des questions dans cette phase de configuration visant à déterminer la marque des produits préférés par l'utilisateur, la fréquence moyenne d'utilisation des produits, la récurrence avec laquelle l'utilisateur veut être conseillé par l'application...

Aussi, l'utilisateur peut réguler la fréquence des messages envoyés/affichés par l'application et l'utilisateur peut également définir des seuils au-dessus duquel il veut toujours être averti, par exemple lorsque la pollution par particules fines dépasse un seuil prédéfini.

L'invention permet ainsi de proposer des produits particulièrement performants pour l'utilisateur en fonction de deux mesures distinctes, une mesure ponctuelle et une mesure effectuée au cours du temps afin d'évaluer le stress subi par la peau de l'utilisateur lors de la journée.

L'invention est définie par les revendications 1-10.

## Revendications

1. Système (10) d'analyse des propriétés physico-chimiques d'une surface cutanée, comportant:
▪ au moins un capteur de contact (12) destiné à venir sur ladite surface cutanée à analyser afin de déterminer une information ponctuelle (20), une à deux fois par jour, relative à une zone particulière de la surface cutanée,
▪ au moins un capteur d'environnement (13) configuré pour accompagner un utilisateur au cours de la journée et mesurer au cours de la journée au moins un paramètre extérieur (16) agissant sur ladite surface cutanée,
▪ une unité de traitement (11) interfacée avec les capteurs (12-13) de contact et d'environnement, ladite unité (11) étant équipée de moyens d'analyse (23) permettant la détermination de certaines propriétés physico-chimiques (24) de la surface cutanée à analyser, à partir des signaux (16, 20) élaborés par des mesures ponctuelles de ladite surface cutanée obtenues par ledit au moins un capteur de contact (12) et des mesures au cours du temps obtenues par ledit au moins un capteur d'environnement (13) ;
characterisé en ce que lesdites mesures ponctuelles correspondent à des mesures effectuées manuellement par un utilisateur avec des intervalles de temps non prédictifs et variables;
lesdites mesures au cours du temps correspondent à des mesures effectuées automatiquement avec des intervalles de temps sensiblement réguliers.

2. Système d'analyse selon la revendication 1, **dans lequel** l'au moins un capteur de contact (12) est configuré pour mesurer un taux d'hydratation et/ou une quantité de sébum et/ou un taux de desquamation.

3. Système d'analyse selon la revendication 1 ou 2, **dans lequel** l'au moins un capteur d'environnement (13) est configuré pour mesurer une température et/ou une information climatique et/ou une information relative à une pollution environnementale.

4. Système d'analyse selon l'une des revendications 1 à 3, **dans lequel** la mesure au cours du temps d'au moins un paramètre extérieur (16) agissant sur ladite surface cutanée est effectuée par une mesure de la position du capteur d'environnement (13), ledit paramètre extérieur (16) étant déterminé par une information climatique en fonction de la position dudit capteur d'environnement (13).

5. Système d'analyse selon l'une des revendications 1 à 4, **dans lequel** l'au moins un capteur de contact (12) comporte des moyens de communication sans fil (19) avec ladite unité de traitement (11).

6. Système d'analyse selon l'une des revendications 1 à 5, **dans lequel** l'au moins un capteur d'environnement (13) comporte des moyens de communication sans fil (15) avec ladite unité de traitement (11).

7. Système d'analyse selon les revendications 5 et 6, **dans lequel** les capteurs (12-13) de contact et d'environnement sont incorporés dans un même boîtier.

8. Système d'analyse selon l'une des revendications 1 à 7, **dans lequel** l'unité de traitement (11) est intégrée dans un téléphone intelligent.

9. Système d'analyse selon l'une des revendications 1 à 8, **dans lequel** l'unité de traitement (11) effectue un classement (25) de la surface cutanée à analyser dans une catégorie prédéterminée (29) en fonction des propriétés physico-chimiques déterminées (24).

10. Système d'analyse selon la revendication 9, **dans lequel** l'unité de traitement (11) effectue une détection d'un produit à conseiller (27) en fonction de la catégorie prédéterminée (29) et d'une base de données de produits traitants (32).

## Patentansprüche

1. System (10) zur Analyse der physikalisch-chemischen Eigenschaften einer Hautoberfläche,
umfassend:
▪ mindestens einen Kontaktsensor (12), zum Aufsetzen auf die zu analysierende Hautoberfläche, um ein- oder zweimal täglich spezifische Informationen (20) über einen bestimmten Bereich der Hautoberfläche zu ermitteln;
▪ mindestens einen Umgebungssensor (13), der dafür ausgelegt ist, einen Benutzer den ganzen Tag über zu begleiten und über den Tag hinweg mindestens einen externen Parameter (16) zu messen, der auf diese Hautoberfläche einwirkt;
▪ eine Verarbeitungseinheit (11), die mit den Kontakt- und Umgebungssensoren (12-13) verbunden ist, wobei diese Einheit (11) mit Analysemitteln (23) ausgestattet ist, die die Bestimmung verschiedener physikalisch-chemischer Eigenschaften (24) der zu analysierenden Hautoberfläche anhand von Signalen (16, 20) ermöglichen, die Ergebnis der spezifischen Messungen dieser Hautoberfläche durch den mindestens einen Kontaktsensor (12) und der Messungen über die Zeit hinweg durch den mindestens einen Umgebungssensor (13) sind;
**dadurch gekennzeichnet, dass** diese spezifischen Messungen Messungen entsprechen, die manuell von einem Benutzer nach nicht vorhersehbaren und variablen Zeitintervallen durchgeführt werden;
die erwähnten Messungen über die Zeit hinweg Messungen entsprechen, die automatisch in im Wesentlichen regelmäßigen Zeitintervallen durchgeführt werden

2. Analysesystem nach Anspruch 1, **bei dem** der mindestens eine Kontaktsensor (12) zur Messung eines Befeuchtungsgrades und/ oder einer Sebummenge und/ oder eines Abschälgrades ausgelegt ist.

3. Analysesystem nach Anspruch 1 oder 2, **bei dem** der mindestens eine Umgebungssensor (13) zur Messung einer Temperatur und/ oder einer Klimainformation und/ oder einer Information über eine Umweltverschmutzung ausgelegt ist.

4. Analysesystem nach einem der Ansprüche 1 bis 3, **bei dem** die Messung über die Zeit hinweg mindestens eines äußeren Parameters (16), der auf diese Hautoberfläche wirkt, durch eine Messung der Position des Umgebungssensors (13) erfolgt, dieser äußere Parameter (16) wird dabei durch eine Klimainformation entsprechend der Position dieses Umgebungssensors (13) bestimmt.

5. Analysesystem nach einem der Ansprüche 1 bis 4, **bei dem** der mindestens eine Kontaktsensor (12) Mittel zur drahtlosen Kommunikation (19) mit der erwähnten Verarbeitungseinheit (11) enthält.

6. Analysesystem nach einem der Ansprüche 1 bis 5, **bei dem** der mindestens eine Umgebungssensor (13) Mittel zur drahtlosen Kommunikation (15) mit der erwähnten Verarbeitungseinheit (11) enthält.

7. Analysesystem nach den Ansprüche 5 und 6, **bei dem** die Kontakt- und Umgebungssensoren (12- 13) im selben Gehäuse untergebracht sind.

8. Analysesystem nach einem der Ansprüche 1 bis 7. **bei dem** die Verarbeitungseinheit (11) in einem Smartphone integriert ist.

9. Analysesystem nach einem der Ansprüche 1 bis 8. **bei dem** die Verarbeitungseinheit (11) eine Einstufung (25) der zu analysierenden Hautoberfläche in einer vorher festgelegten Kategorie (29), in Abhängigkeit von den festgestellten physikalisch-chemischen Eigenschaften (24), vornimmt.

10. Analysesystem nach Anspruch 9, **bei dem** die Verarbeitungseinheit (11) eine Suche nach einem empfehlenswerten Produkt (27) entsprechend der vorher festgelegten Kategorie (29) und einer Datenbank mit Pflegeprodukten (32) durchführt.

## Claims

1. A system (10) for analyzing physico-chemical properties of a skin surface, comprising:
▪ at least one contact sensor (12) for applying to said skin surface to be analyzed in order to determine a one-off information (20), once or twice a day, relating to a particular area of the skin surface,
▪ at least one environment sensor (13) designed to accompany a user throughout the day and to measure, throughout the day, at least one external parameter (16) acting on said skin surface,
▪ a processing unit (11) interfaced with the contact and environment sensors (12-13), said unit (11) being fitted with analysis means (23) allowing the determination of some physico-chemical properties (24) of the skin surface to be analyzed, from signals (16, 20) generated by one-off measurements of the skin surface obtained by the at least one contact sensor (12) and from measurements over time obtained by the at least one environment sensor (13);
***characterized in that***
said one-off measurements correspond to a measurement carried out manually by the user with non-predictable and variable time intervals; said measurements over time correspond to a measurement carried out automatically with almost periodic time intervals.

2. The analysis system according to claim 1, wherein the at least one contact sensor (12) is configured to measure a hydration level and/or a sebum quantity and/or a desquamation level.

3. The analysis system according to claim 1 or 2, wherein the at least one environment sensor (13) is configured to measure a temperature and/or climatic information and/or information relating to environmental pollution.

4. The analysis system according to one of claims 1 to 3, wherein the measurement throughout time of at least one external parameter (16) acting on said skin surface is carried out by a measurement of the position of the environment sensor (13), said external parameter (16) being determined by climatic information as a function of the position of said environment sensor (13).

5. The analysis system according to one of claims 1 to 4, wherein the at least one contact sensor (12) has wireless communication means (19) with said processing unit (11).

6. The analysis system according to one of claims 1 to 5, wherein the at least one environment sensor (13) has wireless communication means (15) with said processing unit (11).

7. The analysis system according to claims 5 and 6, wherein the contact and environment sensors (12-13) are incorporated into a same casing.

8. The analysis system according to one of claims 1 to 7, wherein the processing unit (11) is integrated into a smartphone.

9. The analysis system according to one of claims 1 to 8, wherein the processing unit (11) carries out a classification (25) of the skin surface to be analyzed into a predetermined category (29) according to the determined physico-chemical properties (24).

10. The analysis system according to claim 9, wherein the processing unit (11) carries out a detection of a product to be recommended (27) according to the predetermined category (29) and from a database of treatment products (32).
